# EUROPEAN PATENT APPLICATION

(11) **EP 4 290 232 A1**
(43) Date of publication of application: **13.12.2023**
(21) Application number: 22020265.9
(22) Date of filing: 08.06.2022
(51) Int. Cl.: G01N 33/00, B23K 9/16, B23K 10/00, B23K 26/12, B23K 26/14

(54) **AUTOMATED MONITORING OF GAS QUALITY**

(71) Applicant: Linde GmbH, 82049 Pullach (DE)
(72) Inventor: Bern, Robert, 82049 Pullach (DE)
(74) Representative: Lu, Jing

(57) **Abstract**

The present invention relates to a system (1) for automated analysis of a gas (G) at full operating gas volume flow as used during a joining process or cutting process is disclosed, the system (1) comprising: a torch (2) for conducting the joining or cutting process, the torch (2) comprising a torch head (20), the torch head (20) comprising a gas nozzle (3) being configured to discharge said gas (G), a gas analyzing unit (4) configured to analyze the gas (G) with respect to at least one impurity and/or at least one concentration of a component of the gas (G), wherein the gas analysing unit (4) comprises a connection element (40) comprising an opening configured to accommodate the torch head (20) of the torch (2) so that a sealed flow connection is formed between the gas nozzle (3) and the connection element (40).

## Description

The present invention relates to a system and a method for automated monitoring of gas quality, particularly for a joining process (e.g. welding or brazing process) or cutting process (such as plasma cutting). The joining process can e.g. be gas metal arc welding (GMAW), tungsten inert gas welding (TIG), plasma welding, laser cladding laser welding, plasma brazing, arc brazing, plasma spraying.

Gas analysis is frequently used in order to make sure that gas compositions used in a process (e.g. as shielding gas) are up to the desired specification. Corresponding gas analyzing devices can comprise the capability of remote data transmission, data recording, alerting, and remote access.

Up to now, only random samples have been taken at the gas nozzles used in welding processes during troubleshooting. However, an automated and standardized measuring method is missing.

Particularly, when joining (e.g. by welding or brazing) using e.g. the above-stated procedures, welding defects can occur due to contamination of the gases. Even if the delivery condition of the gases is proper, the downstream components such as pressure regulators, gas lines, hose lines, wire liner, torch cooling, gaskets, fittings, connections, gas mixers, can lead to contamination of the gases.

Often, there is no qualification of the used gas composition before processing (joining or cutting). This is more critical after a period of interrupted work such as overnight, over the weekend or a long production break, e.g., due to holidays.

Possible causes for welding or brazing defects are typically moisture, oxygen and nitrogen that can escape through diffusion-open gas hoses or seals and can get into the gas line system after longer periods of downtime.

Furthermore, leaks in the torch cooling system can cause welding defects. Depending on the exit velocity and given gas flows, gas can be discharged or air can be sucked in along the welding wire core. Further, improper hose connections and leaks may generally lead to welding defects, as well as defective gas mixers or incorrectly set pressures.

In the EN ISO 14175 standard, the gas purities and mixing accuracies are described as delivered, but not at the time of consumption or the point of use. The time of consumption is when the gas flows through the gas nozzle of the respective welding torch / apparatus, affecting the welding process. The point of use refers to the critical location of the gas shielding of the arc from the atmosphere (the process zone).

Based on the above it is an object of the present invention to provide an improved system and method for automated analysis and monitoring of a gas used in a joining or cutting process.

This problem is solved by a system having the features of claim 1 and a method having the features of claim 12. Preferred embodiments of these aspects of the present invention are stated in the sub claims and are described below.

According to claim 1, a system is disclosed for automated analysis and monitoring of a gas, preferably at full operating gas volume flow as used during the joining process (e.g. welding or brazing process) or cutting process, or at a fraction of said full operating gas volume flow, the system comprising:
- a torch for conducting the joining or cutting process, the torch comprising a torch head, the torch head comprising a gas nozzle being configured to discharge said gas,
- a gas analyzing unit configured to analyze the gas with respect to at least one impurity and/or at least one concentration of a component of the gas, wherein the gas analysing unit comprises a connection element comprising an opening configured to accommodate the torch head of the torch so that a sealed flow connection is formed between the gas nozzle and the connection element.

Particularly, the gas is discharged by the gas nozzle on the workpiece(s) to be joined (by welding or brazing) serves to protect the weld pool/molten material from oxidation, but may also be used for other purposes.

Particularly, according to an embodiment, the joining process can be one of: a welding process, a brazing process, gas metal arc welding (GMAW), tungsten inert gas welding (TIG), plasma welding, plasma spraying, plasma brazing, arc brazing. Particularly, the cutting process can be plasma cutting.

Further, particularly, the impurities considered for welding defects, which depend on the material, can be chemical agents such as H₂O, O₂, N₂, CO₂. Furthermore, concentrations of e.g. CO₂, O₂, He, H₂, N₂ in the gas can be too low or too high. There is the option to extrapolate N₂ over the detected O₂ content, if O₂ is not a basic component of a gas being used. If atmospheric gas is leaking into the system and an oxygen contamination is measured, one may be able to approximation the contamination of the N₂ in a similar ratio as oxygen is to N₂ in air - ie.3.7 times more N₂ contamination than oxygen contamination. These possible impurities and also the mixing accuracy (e.g. concentration of the respective component) are preferably measured with a real-time gas analyzing unit in the vicinity of the gas nozzle of the torch.

According to an embodiment, the system is configured not to start the joining or cutting process until the respective impurity is below a predefined limiting value. Furthermore, particularly, said joining process (e.g. welding or brazing processes) and cutting processes are also only started if the gas has its component's concentrations within the specified range.

According to a preferred embodiment of the invention, the gas analyzing unit is configured to analyze the gas with the actual gas volume flow as it is used in a joining or cutting process, particularly to ensure any leaks are properly scaled to the actual flow rate being used in the process. This gas volume flow is denoted as full operating gas volume flow. No special extraction of a low flow from the torch to the gas analyzing unit is required, as currently available gas analyzing units can only handle a small amount of gas flow and special extraction is required. Particularly, a small leak in a low flow process has a larger impact compared to a high flow process. Therefore, in the context of the present invention, the analysis is preferably done at full operating gas volume flow of the gas as being used during an actual joining or cutting process, i.e., the full operating gas volume flow is fed to the gas analysing unit via said connection element, so that the gas analyzing unit can properly analyze the contents. Feeding the full intended operating gas volume flow also has the benefit of allowing to capture any leaks or impurities that make it into the system (through any of the sub-components throughout the joining or cutting process) more easily. Internally, the gas analyzing device can extract a fraction of the gas volume flow needed for the respective sensor. However, the present invention also applies to situations where not the full operating gas volume flow is used, but a fraction of the full operating gas volume flow, particularly at least 10%, particularly at least 20%, particularly at least 30%, particularly at least 40%, particularly at least 50%, particularly at least 60%, particularly at least 70%, particularly at least 80 %, particularly at least 90%.

Particularly in the framework of the present invention, the analysis of the gas is preferably conducted before the joining or cutting process is started and/or after it is completed.

Further, in an embodiment, the connection element comprises one of: at least one seal for contacting the torch head, multiple seals for contacting the torch head, multiple contacts to the torch head on a single sealing surface (so as to prevent atmospheric gases from entering the measurement zone).

Particularly, sealing can be achieved by a force pressing on one or several flexible seals (such as rubber or sealing foams), or by clamping the torch head through the respective seal.

According to yet another embodiment, the at least one seal is configured to seal a face side of the torch head.

Further, according to an embodiment, the connection element comprises a filter, particularly so as to prevent impurities (e.gl such as dust, oils, etc.) from entering the gas analysing unit.

According to a further embodiment of the present invention, the system comprises a power source electrically connectable to the torch, e.g. for allowing the torch to generate an arc, wherein the gas analyzing unit is integrated into the power source, particularly into a housing of the power source, so that both the analyzing unit and the power source share a common housing. Integrating the gas analysing unit into the power source ensures the reduction of needed infrastructures for powering the sensors, extracting the data, processing the data and decision making (starting or stopping the process) as well as porting/loading the data into a Quality Management System (QMS) with time stamping for traceability.

According to a preferred embodiment of the system, the system comprises a control unit operatively connected to the analyzing unit, wherein the control unit is configured to initiate the joining or cutting process in case said at least one impurity is below a predefined limiting value and/or said concentration is within a desired range; and/or wherein the control unit is configured to initiate a purging of the gas nozzle in case said at least one impurity is above a predefined limiting value and/or said concentration is outside a desired range.

According to yet a further embodiment of the system according to the present invention, the system comprises a robotic arm configured to move the torch to the gas analyzing unit so as to automatically establish said flow connection and/or to move the torch to a working site, particularly a welding, brazing or cutting location, when the control unit has received said output signal.

Furthermore, according to an embodiment of the system, the latter is configured to purge the torch by discharging said gas through the gas nozzle for a predefined amount of time in case the processing unit has been idle for more than a predefined idle time.

According to yet another aspect, a method for automated analysis and monitoring of a gas, preferably at full operating gas volume flow as used during a joining or cutting process, or at a fraction of said full operating gas flow, is disclosed, the method comprising the steps of:
- providing a gas analyzing unit,
- providing a control unit and a torch for conducting the joining or cutting process, the torch comprising a gas nozzle being configured to discharge said gas, the torch being further configured to discharge the gas during said joining or cutting process at said full operating gas volume flow,
- providing a flow connection between the torch and the gas analyzing unit and allowing passing of said gas at said full operating gas volume flow or at said fraction of the full operating gas flow to the gas analyzing unit,
- analyzing the gas with the gas analyzing unit with respect to at least one impurity contained in the gas and/or at least one concentration of a component of the gas, wherein an output signal is automatically generated and transmitted to the control unit to allow the control unit to conduct the welding or brazing process in case said at least one impurity is below a predefined limiting value and/or said concentration is within a desired range.

Furthermore, according to an embodiment of the method, the step of providing a flow connection further comprises:
- automatically moving the torch to the gas analyzing unit so as to automatically establish said flow connection, and/or
- establishing a flow connection between the gas nozzle and a connection element being connected to the gas analyzing unit, so as to pass the gas at the full operating gas volume flow to the gas analyzing unit via the connection element.

According to a preferred embodiment of the method, the control unit, being operatively connected to the analyzing unit, initiates the joining or cutting process in case said at least one impurity is below a predefined limiting value and/or said concentration is within a desired range; and/or the control unit initiates a purging of the gas nozzle in case said at least one impurity is above a predefined limiting value and/or said concentration is outside a desired range.

According to an embodiment of the method, wherein the system is configured to purge the torch by discharging said gas through the gas nozzle for a predefined amount of time in case the torch (or welding or brazing system) has been idle for more than a predefined idle time.

Further, in an embodiment of the method, the joining process is one of: a welding process, a brazing process, GMAW, TIG, plasma welding, laser cladding, laser welding, plasma spraying, arc brazing, plasma brazing. Furthermore, according to an embodiment of the method, the cutting process is plasma cutting.

Further, in an embodiment of the method, a sealed flow connection is formed between the gas nozzle and the connection element.

According to an embodiment, the flow connection is formed by accommodating the torch head in an opening of the connection element being connected to the gas analysing unit, wherein the opening forms a funnel, particularly so as to be able to accommodate torch heads of different diameter. Particularly, the torch head forms a front element of the torch that can comprise one, several or all of: a nozzle, a shielding nozzle, a shield cap, etc.

Further, in an embodiment, said flow connection is sealed by one of: at least one seal for contacting the torch head, multiple seals for contacting the torch head, multiple contacts to the torch head on a single sealing surface (so as to prevent atmospheric gases from entering the measurement zone, see also above).

Further, in an embodiment, a face side of the torch head is sealed with respect to a face side of the connection element.

Particularly, the gas passed to the gas analyzing unit is guided through at least one filter, particularly so as to prevent impurities (e.gl such as dust, oils, etc.) from entering the gas analysing unit.

Further, according to an embodiment of the method, an arc is generated by the torch with help of a power source, wherein the gas analyzing unit is integrated into the power source.

In the following, embodiments of the present invention as well as further features and advantages shall be explained with reference to the Figures, wherein
- Fig. 1: shows a schematic illustration of an embodiment of the system and method according to the present invention;
- Fig. 2: shows a block diagram of an embodiment of the method according to the present invention;
- Fig. 3: shows further block diagrams relating to purging of the torch according to embodiments of the method according to the present invention;
- Fig. 4: shows an embodiment of a connection element of the system for providing a flow connection between the torch head and the gas analyzing unit;
- Fig. 5: shows a further embodiment of the connection element;
- Fig. 6: shows further embodiment of the connection element with face side sealing with respect to the torch head;
- Fig. 7: shows a modification of the embodiment of the connection element shown in Fig. 6; and
- Fig. 8: shows an embodiment of the system having a gas analyzing unit integrated into a power supply of the system.

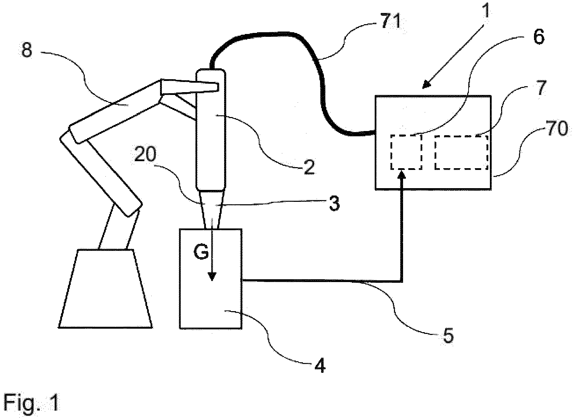

Fig. 1 shows a schematic illustration of a system 1 for automated analysis and monitoring of a gas G used in a joining process (such as a welding or brazing process, or a cutting process, e.g., plasma cutting) at full operating gas volume flow, wherein the system 1 comprises a gas analyzing unit 4, a control unit 6, and a torch 2 for conducting the joining or cutting process, the torch 2 comprising a gas nozzle 3 being configured to discharge said gas G, the torch 2 being further configured to discharge the gas G during said joining or cutting process at said full operating gas volume flow, wherein the torch 2 is configured to be connected to the gas analyzing unit 4 to provide a flow connection between the torch 2 and the gas analyzing unit 4 to allow passing of said gas G at said full operating gas volume flow to the gas analyzing unit 4, wherein preferably the gas analyzing unit 4 is configured to analyze the gas G with respect to at least one impurity and/or at least one concentration of a component of the gas G, wherein the gas analyzing unit 4 is configured to generate an output signal 5 and to transmit the output signal 5 to the control unit 6 allowing the control unit 6 to initiate the joining or cutting process in case said at least one impurity is below a predefined limiting value and/or said concentration is within a desired range. Otherwise, if these conditions are not met, the system 1 may purge the gas nozzle, to remove said impurities.

Particularly, the present invention allows automated measurement and monitoring (qualification) of the gas purity and also the mixing accuracy directly at the gas nozzle 3 for joining processes, particularly welding processes such as GMAW, or arc brazing process at full operating gas volume flow of the torch (also other processes can be used, see above).

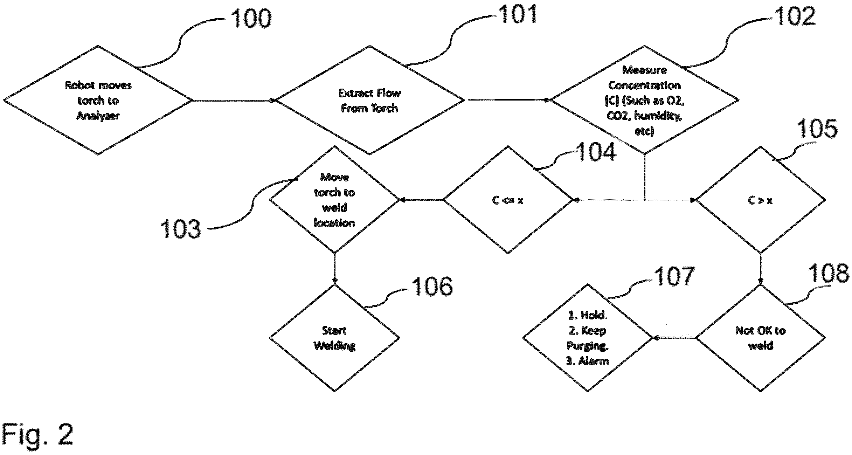

Particularly, the gas analyzing unit 4 is connected to the control unit 6 that is configured to allow providing electric energy to the torch 2 for conducting the welding/brazing process so that the system 1 can start when the measurement shows a certain value. The process / control unit 6 receives the "OK-to-Proceed" signal 5 when the gas analyzing unit 4 detects a stable composition level with the acceptable parameters measured. Fig. 2 illustrates the signal flow and process steps of an embodiment of the method according to the present invention. According thereto, in an embodiment, robot arm 8 comprised by the system 1 moves the torch 2 to the gas analyzing unit 4 (100). The latter extracts a full operating gas volume flow of the gas nozzle 3 (101) and measures a concentration [c] such as O₂, CO₂, humidity etc (102). In case this impurity concentration c is above a certain limit x (105), welding/brazing is not started, but further measures can be performed to reduce impurities, such as purging. An alarm informing about the impurities in the gas G can be issued to the user (107). In case concentration c is below the threshold x (104), the torch 2 is moved to the welding location (105) and welding/brazing is started (106) by the control unit 6.

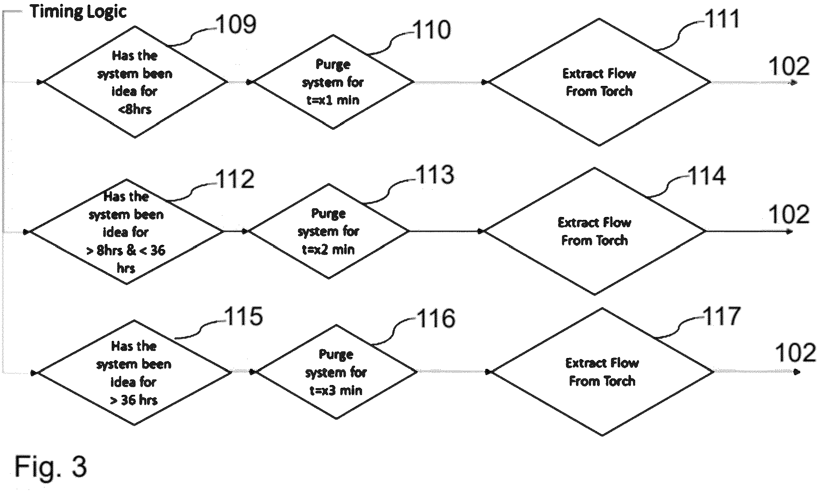

Fig. 3 shows a further embodiment of the method relating to purging of the system 1 in case the system has been idle for a certain time. For this a timing logic is implemented into the system 1 according to which in case the system has been idle for less than 8 hours (109) the system 1 is purged for at least a minute (110). Afterwards gas G is extracted from the torch 2 (111) and the method continues with step 102 shown in Fig. 1.

Furthermore, in case the system 1 has been idle for more than 8 hours but less than 36 hours (112) the system 1 is purged for at least two minutes (113). Afterwards gas G is extracted from the torch 2 (114) and the method continues with step 102 shown in Fig. 1.

Furthermore, in case the system 1 has been idle for more than 36 hours (115) the system 1 is purged for at least three minutes (116). Afterwards gas G is extracted from the torch 2 (117) and the method continues with step 102 shown in Fig. 1.

Particularly, the contaminants considered for welding (or brazing) defects, which depend on the material, are chemical agents such as H₂O, O₂, N₂, CO₂. Further components of the gas may have concentrations of e.g. CO₂, O₂, He, H₂, N₂being too low or too high in the gas mixture. Particularly, there is the option to extrapolate N₂ over the detected O₂ content, if O₂ is not a basic component of the gas. These possible contaminations and also mixing accuracy are preferably measured in real-time with the gas analyzing unit 4 directly at the gas nozzle 3. Joining processes such as welding/brazing processes, or cutting processes are not released until the specifications regarding impurities are met. Further, these processes are also only released if the specified gas mixture is within the specified tolerances.

Preferably, the individual measurement of the gas G is carried out with the actual gas volume flow as it is used in the respective joining process (e.g. welding or brazing process) or cutting process. No special extraction of low flows from the torch 2 to the gas analyzing unit 4 are required, as currently available gas analyzing units can only handle a small amount of gas flow and special extraction is required.

Preferably, the gas G is at least analyzed prior to start-up after a weekend or after a pre-defined shutdown period in order to release a process. Intermediate measurements and measurements after completion of a welding process may also be useful. Joining processes or cutting processes can thus be monitored and subjected to a minimum standard. At present, all decisive welding or brazing parameters can be monitored and documented, but the gas G that enters the process directly through the gas nozzle 3 is not taken into account. However, this gas contributes to the success or failure of a weld.

Possible applications of the joining processes (e.g. welding or brazing processes) or the cutting processes (e.g. plasma cutting) monitored with the system or method according to the present invention can would be pressure pipeline construction, automotive, tank construction, crane construction, bridge construction, formwork construction, ropeway construction etc.

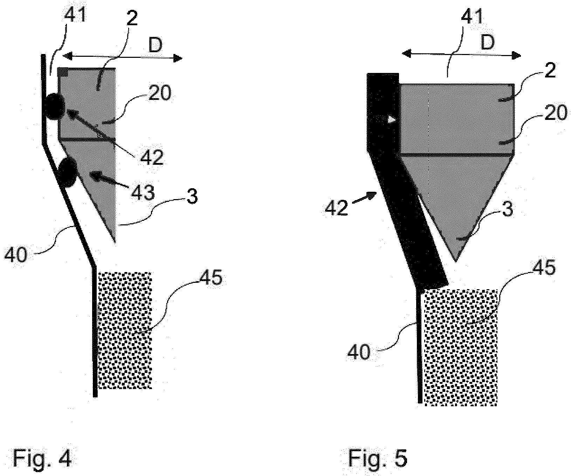

As further shown in Figs. 4 to 7, the system 1 according to the present invention comprise a connection element 40 that is preferably configured to accommodate multiple torch heads or guns, burners, etc. The connecting element 40 may be connected to accommodate a variety of torch heads / nozzles including GMAW, GTAW, PAW, laser welding, plasma spraying, laser cladding etc., so that a seal is formed between the torch head 20 and the connecting element 40.

For example, as shown in Fig. 4, the connecting element 40 can comprise a funnel-shaped opening 41 and multiple seals 42, 43, to provide multiple contact sealing lines to prevent atmospheric gases from entering the measurement zone. Alternatively, as shown in Fig. 5, a single seal / sealing surface may be provided in the funnel-like opening 41 of the connection element 40. The respective connection element 40 can comprises at least one built-in filter 45 to prevent process dust, oils, etc. from entering the gas analyzing unit 4 without clogging the system 1.

Due to the funnel-shape of the connection element 40, the system 1 can accommodate multiple torch designs (head 20) and provide a tight transition.

Furthermore, the connection element 40 may provide a multiple sealing mechanism so that sealing is performed on multiple surfaces or multiple times on a single surface. Sealing can thereby be achieved by a force pressing on flexible and sealing elements (such as rubber or sealing foams), or by clamping through the sealing rubber/foam onto the respective torch head 20.

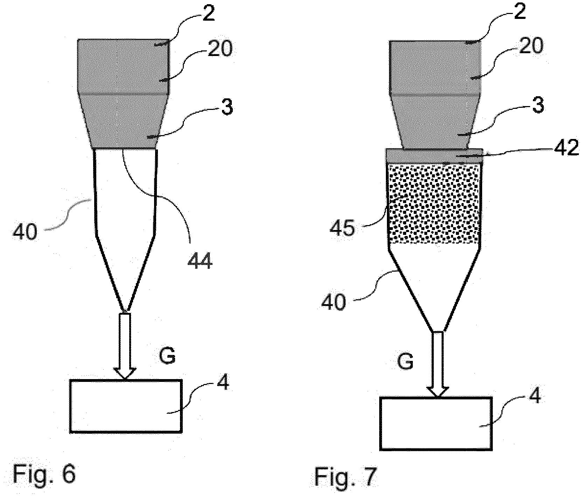

Instead of providing a sealing surface or seal(s) in the internal space of the connection element 40 as shown in Figs. 4 and 5, also the face side of the connection element / torch head can be sealed by an appropriate seal 44 (cf. Fig. 6). Further, as shown in Fig. 7 a seal 42 (e.g., O-ring) can be provided along the circumference of the opening 41 so that the torch head 20 can be inserted into central opening of the O-ring 42 to seal the flow connection between torch head 20 and connection element 40.

Generally, the system 1 can be integrated into robotic and mechanized applications where the torch is automatically translated to the gas analyzing system 4 for analysis.

Particularly, the extraction and analysis of the gas G may be done at one of multiple stages of the process such as before starting the process or after the processing (welding or brazing) has been completed. The extraction and analysis may also be done during the process. Remedies maybe implemented to activate purge sequence to bring lower the contamination. The purge sequence may be increased progressively.

The extraction and analysis may be continuous or may be set at given intervals. The intervals may vary and can be a function of the process itself, the production time or other factors.

These torches 2 may be torches 2 that are configured for conducting the joining processes mentioned herein (e.g. welding processes, brazing processes, GMAW, TIG, plasma welding, plasma spraying, plasma cutting, arc brazing, plasma brazing etc.) or cutting process (such as plasma cutting).

In particularly, the gas G extracted via the respective connection element 40 represents a true sample of the gas G. It will include any and all cumulative impurities introduced at all stages in the system 1 (from the supply line, to the connections to the process itself, the gas control unit, the connection of a torch lead 71 to the power source 7 and gas control unit up to the torch head 20 itself (see also Fig. 1).

The gas sample is preferably extracted when the process is flowing gas G at its recommended gas volume flow so as to properly measure the concentrations of all the components of the gas G and the impurities therein. Therefore, extracting from the critical location that is flowing too little or too high of a flow may overestimate or underestimate the concentrations and therefore provide erroneous input for the decision-making process (this being either done by the welder or by an automated system in mechanized or robotic applications). Further, preferably, all the data is analyzed and recorded with a time stamp. Therefore, the gas composition conforms to the ISO 14175 and ISO 3834.

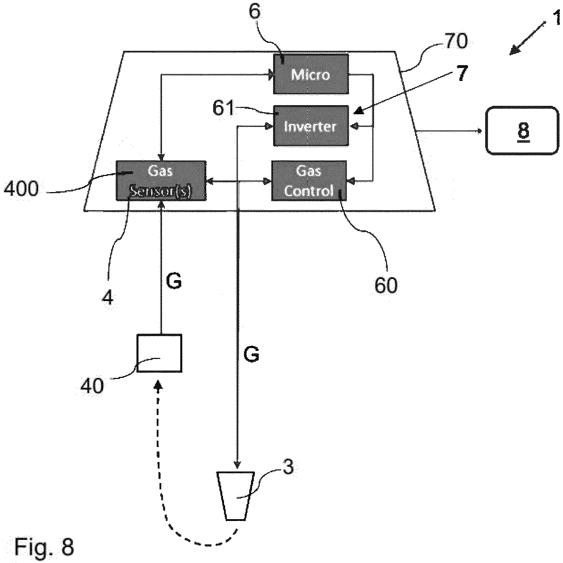

Further, Fig. 8 illustrates a further embodiment of a system 1 according to the present invention. Here, the gas analyzing unit 4, and particularly its gas sensors, are integrated into a housing 70 of the system 1 that also accommodates a power source 7 to provide electrical energy to the joining or cutting process, thus ensuring the reduction of needed infrastructures for powering the sensors, extracting the data, processing the data and decision making (starting or stopping the process) as well as porting/loading the data into a Quality Management System (QMS) 8 with time stamping for traceability. This significantly reduces the infrastructure cost and the cost in developing interfaces and communication protocols to communicate with the power supply and the quality management system.

Particularly, the housing 70 of the power source 7 houses the sensors 400 of the gas analyzing unit 4, wherein the power source 7 provides the needed power to energize the sensors 400 and the sensor subcomponents. The sensors 400 may be oxygen concentration sensors, CO₂ infrared sensors, humidity sensors, MEMS (micro electro mechanical systems), hydrogen sensors or others. The subcomponents of the sensor(s) 400 may include small flow meters/controllers, pressure and temperature sensors and others.

Further, the system comprises a micro-controller 6 embedded in the housing 70 of the power supply 7 to provide a control logic for running and/or coordinating not only an inverter power module 61 of the power supply 7 and a gas controller 60 for controlling gas volume flow of the gas G, but also the embedded sensors 400 of the gas analyzing unit 4 (alternatively single gas sensors).

Furthermore, within the scope of this invention is having a unitary body module that houses the gas sensors 400 embedded in it and intended to fit (mechanically and electrically) within the power source's 7 housing 70 or being attached to the power source 7 as a submodule. The attachment may be internal or external.

Within the scope of this invention is having the single sensor 400 or multiple sensors 400 installed in the housing 70 of the power source 7 natively and not being housed in their own unitary body. The gas sensors 400 or the unitary body of the gas analyzing unit 4 can be brought in fluid communication with the torch 2 being used (i.e. welding torch, cutting torch, spraying torch, etc.). This may be achieved via the connection element 40.

The data collected from the gas sensors 400 may be saved to an on-board memory storage device or directly uploaded to a server or the cloud through ethernet LAN, WIFI, cellular network or other means.

Particularly, the data collected from the gas sensors 400 are time-stamped and synchronized with the native data collected from the process such as the voltage, current and gas volume flows as well as the wire feed speeds and other signals that may be integrated into the power source 7 (i.e. welding speed).

The data may also be ported to an external server or cloud service through the main power supply interface protocols and implemented into the manufacturing quality management system [QMS] 8 for compliance with ISO 14175, ISO 3834 and ISO 1090.

## Claims

**1.** A system (1) for automated analysis of a gas (G), preferably at full operating gas volume flow as used during a joining or cutting process, or at a fraction of said full operating gas volume flow, comprising:
- a torch (2) for conducting the welding or brazing process, the torch (2) comprising a torch head (20), the torch head (20) comprising a gas nozzle (3) being configured to discharge said gas (G),
- a gas analyzing unit (4) configured to analyze the gas (G) with respect to at least one impurity and/or at least one concentration of a component of the gas (G),
wherein the gas analysing unit (4) comprises a connection element (40) comprising an opening (41) configured to accommodate the torch head (20) of the torch (2), so that a sealed flow connection is formed between the gas nozzle (3) and the connection element (40).

**2.** The system according to claim 1, wherein the opening (41) forms a funnel, particularly so as to accommodate torch heads (20) of different diameter (D).

**3.** The system according to claim 1 or 2, wherein the connection element (40) comprises one of: at least one seal (42, 43) for contacting the torch head (20), multiple seals (42, 43) for contacting the torch head (20), multiple contacts to the torch head on a single sealing surface.

**4.** The system according to claim 3, wherein the at least one seal (44) is configured to seal a face side of the torch head (20).

**5.** The system according to one of the claims 1 to 4, wherein the connection element (40) comprises a filter (45), particularly so as to prevent impurities from entering the gas analysing unit (4).

**6.** The system according to one of the preceding claims, wherein the system (1) comprises a power source (7) comprising a housing, the power source being electrically connectable to the torch (2) for allowing the torch (2) to generate an arc, wherein the gas analyzing unit (4) is integrated into the housing (70).

**7.** The system according to one of the preceding claims, wherein the system comprises a control unit (6) operatively connected to the analyzing unit (4), wherein the control unit (6) is configured to initiate the joining or cutting process in case said at least one impurity is below a predefined limiting value and/or said concentration is within a desired range; and/or wherein the control unit (6) is configured to initiate a purging of the gas nozzle (3) in case said at least one impurity is above a predefined limiting value and/or said concentration is outside a desired range.

**9.** The system according to one of the preceding claims, wherein the system comprises a robotic arm (8) configured to move the torch (2) to the gas analyzing unit (4) so as to automatically establish said flow connection and/or to move the torch (2) to a welding or brazing location when the control unit (6) has received said output signal (5).

**10.** The system according to one of the preceding claims, wherein the system (1) is configured to purge the torch (2) by discharging said gas (G) through the gas nozzle (3) for a predefined amount of time in case the processing unit (6) has been idle for more than a predefined idle time.

**11.** The system according to one of the preceding claims, wherein the joining process is one of: a welding process, a brazing process, GMAW, TIG, plasma welding, plasma spraying; and/or wherein the cutting process is plasma cutting.

**12.** A method for automated analysis of a gas (G), preferably at full operating gas volume flow as used during a joining or cutting process, or at a fraction of said full operating gas volume flow, the method comprising the steps of:
- providing a gas analyzing unit (4),
- providing a control unit (6) and a torch (2) for conducting the joining or cutting process, the torch (2) comprising a gas nozzle (3) being configured to discharge said gas (G), the torch (2) being further configured to discharge the gas (G) during joining or cutting,
- providing a flow connection between the torch (2) and the gas analyzing unit (4) and allowing passing of said gas (G) to the gas analyzing unit (4) at said full operating gas volume flow or at said fraction of the full operating gas flow.
- analyzing the gas (G) with the gas analyzing unit (4) with respect to at least one impurity and/or at least one concentration of a component of the gas (G), wherein an output signal (5) is automatically generated and transmitted to the control unit (6) to allow the control unit (6) to conduct the joining or cutting process in case said at least one impurity is below a predefined limiting value and/or said concentration is within a desired range.

**13.** The method according to claim 12, wherein the step of providing a flow connection further comprises
- automatically moving the torch (2) to the gas analyzing unit (4) so as to automatically establish said flow connection, and/or
- establishing a flow connection between the gas nozzle (3) and a connection element (40) being connected to the gas analysing unit (4), so as to pass gas to the gas analyzing unit (4) via the connection element (40).
